(19) Europäisches Patentamt
European Patent Office
Office européen des brevets

(11) Veröffentlichungsnummer : **0 176 716**
**B1**

(12) # EUROPÄISCHE PATENTSCHRIFT

(45) Veröffentlichungstag der Patentschrift : **25.05.88**

(51) Int. Cl.⁴ : **C 07 D499/70**, C 07 D499/16, A 61 K 31/43// C07D307/54, C07D409/14

(21) Anmeldenummer : **85110189.9**

(22) Anmeldetag : **14.08.85**

(54) **Kristallines Natriumsalz der D-6-(alpha-[(2-Oxo-3-furfuryliden-amino-imidazolidin-1-yl)carbonylamino]-thienyl-2-acetamido)-penicillansäure, Verfahren zur Herstellung und seine Verwendung in Arzneimitteln.**

(30) Priorität : **25.08.84 DE 3431273**

(43) Veröffentlichungstag der Anmeldung : **09.04.86 Patentblatt 86/15**

(45) Bekanntmachung des Hinweises auf die Patenterteilung : **25.05.88 Patentblatt 88/21**

(84) Benannte Vertragsstaaten : **AT BE CH DE FR GB IT LI NL SE**

(56) Entgegenhaltungen :
**EP-A- 0 000 392**
**EP-A- 0 000 393**
**CHEMICAL ABSTRACTS, Band 81, Nr.2, 15. Juli 1974, Seite 149, Nr. 6279s, Columbus, Ohio, US**
**CHEMICAL ABSTRACTS, Band 87, Nr. 23, 5. Dezember 1977, Seite 611, Nr. 184490k, Columbus, Ohio, US**
**CHEMICAL ABSTRACTS, Band 88, Nr. 1, 2. January 1978, Seite 587, Nr. 6875c, Columbus, Ohio, US**
**CHEMICAL ABSTRACTS, Band 87, Nr. 21, 21. November 1977, Seite 582, Nr. 168073w, Columbus, Ohio, US; M.V. USACHII et al.: "Sodium salt of benzylpenicillin"**
**CHEMICAL ABSTRACTS, Band 101, Nr. 3, 16. Juli 1984, Seite 577, Nr. 23222s, Columbus, Ohio, US**

(73) Patentinhaber : **BAYER AG**
**Konzernverwaltung RP Patentabteilung**
**D-5090 Leverkusen 1 Bayerwerk (DE)**

(72) Erfinder : **König, Hans-Bodo, Dr.**
**Herberts-Katernberg 10**
**D-5600 Wuppertal 1 (DE)**
Erfinder : **Metzger, Karl Georg, Dr.**
**Pahlkestrasse 75**
**D-5600 Wuppertal 1 (DE)**
Erfinder : **Schröck, Wilfried, Dr.**
**Pahlkestrasse 33**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 176 716 B1

Jouve, 18, rue St-Denis, 75001 Paris, France

**Beschreibung**

Die Erfindung betrifft das Natriumsalz der D-6-{α-[(2-oxo-3-furfuryliden-amino-imidazolidin-1-yl) carbonylamino]-thienyl-2-acetamido}-penicillansäure in reiner kristalliner Form, Verfahren zu dessen Herstellung sowie seine Verwendung in Arzneimitteln.

Im europäischen Patent Nr. 392 ist unter weiteren die Verbindung der Formel (I)

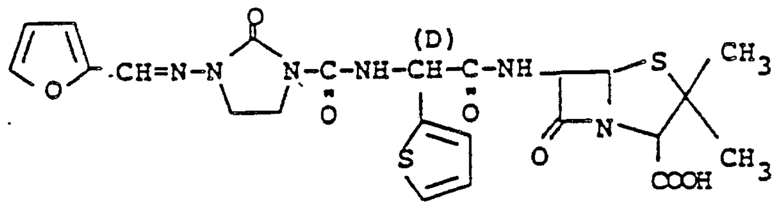

(I)

beschrieben.

Ausgangsstoff für die Synthese dieses Penicillins war die D,L-α-Amino-thienylessigsäure. Durch Umsetzung mit 6-Aminopenicillansäure erhielt man α-Amino-thienyl-2-acetamido-penicillansäure in schlechter Ausbeute (11-31 %). Gleichzeitig trat Diastereomerentrennung ein. Genauere Untersuchungen ergaben ein Diastereomerenverhältnis von D : L = 55-61 : 45-39.

Durch Umsetzen der α-Amino-thienyl-2-acetamido-penicillansäure mit 1-Chlorcarbonyl-2-oxo-3-furfuryliden-amino-imidazolidin erhielt man die Penicillansäure der Formel (I) in reiner kristalliner Form (D : L = 92 : 8).

Zur Gewinnung des Natriumsalzes wurde die Penicillansäure (I) in der berechneten Menge 0,5 n Natronlauge gelöst und diese Lösung lyophilisiert (IR Spektrum in Fig. 1). Dieses durch Gefriertrocknung einer wäßrigen Lösung erhaltene amorphe Natriumsalz ist ansich sehr gut in Wasser löslich. Je nach Herstellungs-Charge bleiben jedoch konzentrierte wäßrige Lösungen (beispielsweise 15 %ige) nur mehr oder weniger kurze Zeit klar und dünnflüssig. Einige gelieren schon nach wenigen Minuten, so daß man die Gefäße auf den Kopf stellen kann, ohne daß etwas herausfließt. Eine Erklärung für dieses unkontrollierbare Verhalten der konzentrierten wäßrigen Lösungen, das eine intravenöse Anwendung stark erschweren oder unmöglich machen würde, gibt es bisher nicht.

Es gelang in zahlreichen Versuchen nicht, durch Kristallisation aus Lösungsmitteln kristallines Na-Salz zu erhalten ; so bildet sich beispielsweise bei der Fällung wäßrig-alkoholischer Lösungen mit Isopropanol amorphes Produkt welches hartnäckig Isopropanol auch im Hochvakuum festhält und dessen Lösungen gelieren (IR Spektrum in Fig. 2).

Überraschenderweise wurde nun gefunden, daß aus wäßrigem Ethanol ein Natriumsalz der Penicillansäure der Formel I in kristalliner Form erhalten wird (IR Spektrum in Fig. 3), das stabil ist, leicht von anhaftendem Alkohol befreit werden kann und dessen Lösungen tagelang klar und dünnflüssig bleiben und nicht gelieren.

Die Erfindung betrifft daher das Natriumsalz der D-6-{α-[(2-Oxo-3-furfurylidenamino-imidazolidin-1-yl)-carbonylamino]-thienyl-2-acetamido}-penicillansäure der Formel (I)

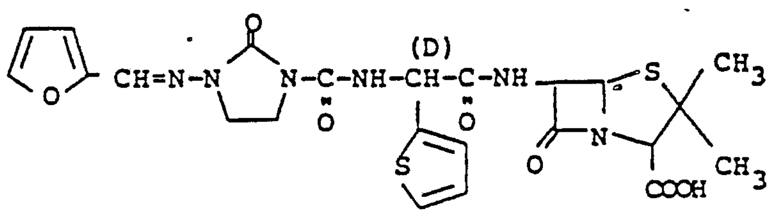

(I)

in reiner kristalliner Form, gekennzeichnet durch NMR Signale bei δ = 9,36 (1H), 9,23 (1H), 7,8 (2H), 7,5 (1H), 7,25 (1H), 7,05 (1H), 6,89 (1H), 6,62 (1H), 6,2 (1H), 5,74 (1H), 5,56 (1H), 4,35 (1H), 3,92 (4H), 1,6 (3H) und 1,5 ppm (3H) (in DMF-$d_7$).

Es wurde außerdem gefunden, daß man das obengenannte Natriumsalz der Penicillansäure der Formel (I) in kristalliner Form erhält, wenn man

(A) das amorphe Natriumsalz der D-Penicillansäure der Formel (I)

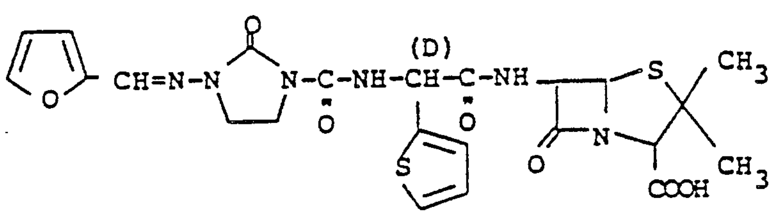

(I)

aus einem Ethanol-Wasser-Gemisch in den Mengenverhältnissen von Ethanol : Wasser im Bereich von 5 : 1 bis 15 : 1 bei Temperaturen von 0 °C bis 50 °C kristallisiert, oder

(B) die kristalline Säure der Formel (I) in Lösung in ihr Natriumsalz überführt und dieses aus Ethanol-Wasser-Gemischen wie unter (A) kristallisiert.

Die Synthese der als Ausgangsstoff verwendeten Penicillansäure der Formel (I) sowie deren Natriumsalz wurde abweichend von der im europäischen Patent Nr. 392 auf einem Weg durchgeführt, der zu einem diastereomer einheitlichen Produkt führt :

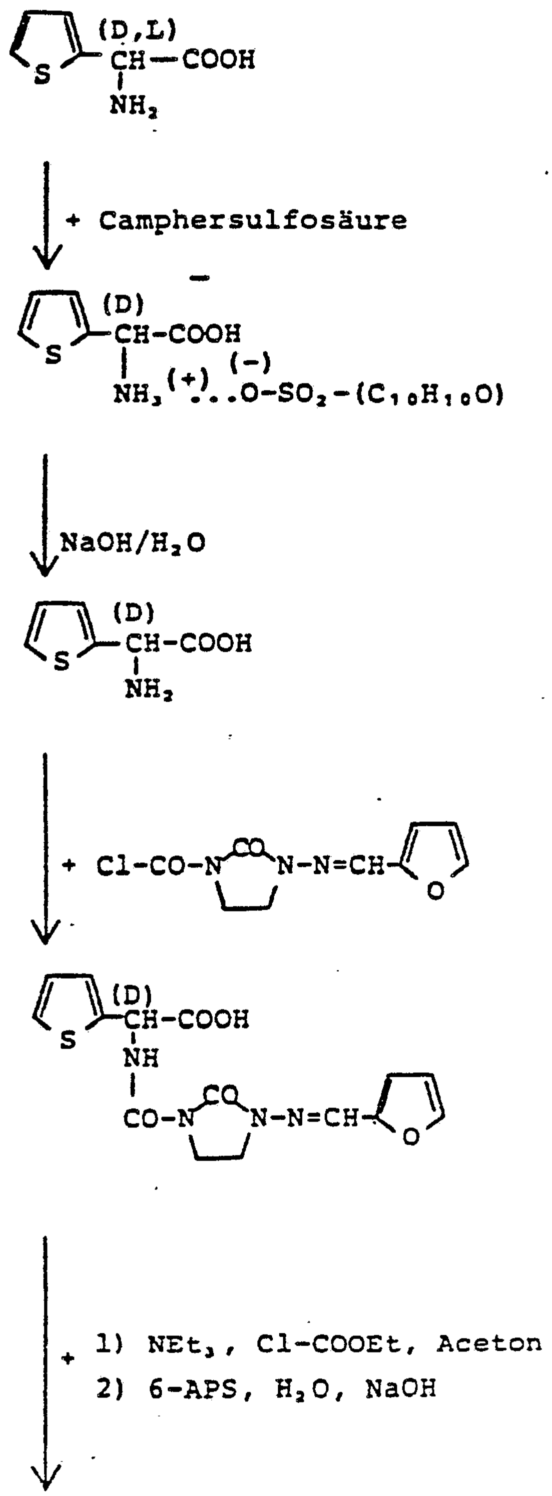

(Fortsetzung)

(I)

NaOH

Natriumsalz von (I)

Die Verfahren werden bevorzugt in einem Temperaturbereich von 20 °C bis 40 °C durchgeführt. Im allgemeinen arbeitet man bei Raumtemperatur.

Die Verfahren können bei Normaldruck oder bei erhöhtem Druck durchgeführt werden. Normalerweise arbeitet man bei Normaldruck.

Das Verhältnis von Menge des Lösungsmittelgemisches zur Menge des gelösten Stoffes kann in einem weiten Bereich variiert werden. Bevorzugt ist pro 0,1 mol gelöstem Stoff eine Menge an Lösungsmittelgemisch von 0,5 bis 3 Liter.

Das kristalline reine Natriumsalz der D-Penicillansäure der Formel (I) ist als Wirkstoff gegen ein breites Spektrum von Mikroorganismen wirksam. Aufgrund seiner Eigenschaft, Lösungen zu bilden, die tagelang klar und dünnflüssig bleiben und nicht gelieren, eignet sich der erfindungsgemäße Wirkstoff besonders zur Herstellung von Lösungen zur Injektion.

Zur vorliegenden Erfindung gehören pharmazeutische Zubereitungen, die neben nicht toxischen, pharmazeutisch geeigneten Trägerstoffen den erfindungsgemäßen Wirkstoff enthalten. Unter nicht toxischen pharmazeutisch geeigneten Trägerstoffen sind flüssige Verdünnungsmittel jeder Art zu verstehen, insbesondere Wasser.

Zur parentralen Applikation kommen die Lösungen auch in steriler und blutisotoner Form vorliegen.

Zur vorliegenden Erfindung gehören auch pharmazeutische Zubereitungen in Dosierungseinheiten. Dies bedeutet, daß die Zubereitungen in Form von Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 der Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder Viertel einer Tagesdosis entspricht.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer dem erfindungsgemäßen Wirkstoff auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des Wirkstoffs mit dem oder den Trägerstoffen.

Die Wirkstoffe oder pharmazeutische Zubereitungen können parenteral wie intravenös oder intramuskulär appliziert werden.

Im allgemeinen hat es sich sowohl in der Human- als auch in der Veterinärmedizin als vorteilhaft erwiesen, den erfindungsgemäßen Wirkstoff in Gesamtmengen von etwa 5 bis 1.000, vorzugsweise 20 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den erfindungsgemäßen Wirkstoff, vorzugsweise in Mengen von Etwa 1 bis etwa 250, insbesondere 10 bis 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, den Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der oben genannten Menge Wirkstoff auszukommen, während in anderen Fällen die oben angeführte Wirkstoffmenge überschritten werden

muß. Die Festlegung der jeweils erforderlichen optimalen Dosierung und Applikationsart des Wirkstoffs kann durch jeden Fachmann aufgrund seines Fachwissens leicht erfolgen.

Fig. 1 zeigt ein IR Spektrum des amorphen Natriumsalzes (lyophil. nach Beispiel 5, EP-A 0 000 392). Auf der X-Achse ist die Wellenlänge in $cm^{-1}$ aufgetragen. Die Zahlen auf der Y-Achse entsprechen der Durchlässigkeit in %. Die Zahlen am oberen Rand des Spektrums entsprechen der Frequenz in mikron. Als Lösungsmittel wurde Nujol verwendet.

Fig. 2 zeigt ein IR-Spektrum des amorphen Natriumsalzes aus i-PrOH (Vergleichsbeispiel 2).

Fig. 3 zeigt ein IR-Spektrum des kristallinen Natriumsalzes aus EtOH (Beispiel 1, 2).

In den Zeichnungen Fig. 2 und Fig. 3 entsprechen die Zahlen an den X- bzw. Y-Achsen den für Fig. 1 angegebenen Einheiten.

Vergleichsbeispiel 1.1

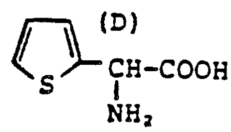 · 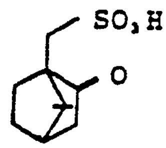

1,142 kg (7,3 M) D,L-α-Thienyl-(2)-glycin und 1,83 kg (7,3 M) (+) Camphersulfonsäurehydrat (CSS techn.) wurden mit 2,03 l Wasser bei 72° Innentemperatur in Lösung gebracht, heiß von geringfügigen Verunrenigungen der CSS abgesaugt, angeimpft und stehen gelassen. Nach 1 Stunde wurde in ein Eisbad gesetzt und bis 10 °C Innentemperatur (ca. 1 Stunde Dauer) weiter abgekühlt. Es wurde abgesaugt, sorgfältig abgepreßt, mit Aceton gewaschen und im Vakuum-Schrank getrocknet.

Ausbeute : 898,9 g (31,7 %).

$[\alpha]_D = -36{,}4°$ in $CH_3OH$.

IR-Banden bei 3 260-2 100, 1 746, 1 699, 1 620, 1 500, 1 200, 1 140, 1 031 und 751 $cm^{-1}$ (in Nujol).

Vergleichsbeispiel 1.2

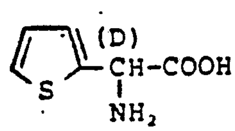

800 g Camphersulfonsäuresalz des D-Thienylglycins aus 1.1 wurden in 2 l Wasser suspendiert und mit 2,5 l i-Propanol verdünnt. Mit 2 n NaOH stellte man pH = 6,0 ein und hielt den pH bei 6 bis er konstant war. Nun verrührte man mit weiteren 2,5 l i-Propanol, rührt 30 Minuten nach, saugt ab und wusch mit i-Propanol und anschließend mit Ether nach. Es wurde 24 Stunden im Trockenschrank bei 50° über $P_2O_5$ getrocknet.

292 g (90 %). 1 n HCl : $[\alpha]_D = -115{,}4°$. IR-Spektrum ist verschieden von dem des racemischen Thienylglycins.

IR-Banden bei 3 250-2 000, 1 600, 1 498, 1 275, 1 122, 904, 850, 719 und 688 $cm^{-1}$ (in Nujol).

Die D = R-Konfiguration der Säure wurde mittels Circulardichroismus-Messung bestätigt.

Vergleichsbeispiel 1.3

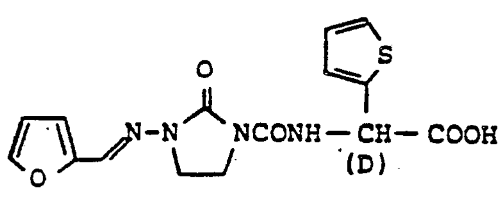

573 g (3,65 Mol) (D)-α-Thienyl-(2)-glycin wurden in 4,5 l $THF/H_2O$ (1 : 1) unter Rühren und äußerer Kühlung mit Eis/Wasser mit Triethylamin*) auf pH 7 gestellt. Unter weiterer Kühlung wurden nun 881 g (3,65 Mol) 1-Chlorcarbonyl-2-oxo-3-furfurylidenamino-imidazolidinon eingetragen und gleichzeitig der pH mit Triethylamin auf 6,5-7,0 gehalten. Die Innentemperatur soll 35° nicht überschreiten. Man rührte bis der pH bei 7 konstant blieb. Man fügte 1,5 l Wasser zu, entfernte das THF im Vakuum-Rotationsverdamp-

*) Verwendet man NaOH als Base, so kristallisiert bei der vorliegenden Konzentration das Na-Salz der Präcursorsäure häufig aus.

fer und rührte die wäßrige Phase 15 Minuten mit 350 g Kieselgur durch. Nun wurde abgesaugt mit ca. 1 l Wasser gewaschen und die vereinigten Filtrate mit 1 n HCl auf pH = 2 eingestellt, nach kurzer Zeit abgesaugt, mit Wasser, dann mit Ethanol und Ether gewaschen und im Trockenschrank bei 60° getrocknet.

Das Produkt (1 121 g, 85 %) (Zers. ≥ 243 °C) enthielt nach dem NMR-Spektrum ca. 1,7 % 1-Furfurylidenamino-2-oxo-imidazolidin, ca. 2 % Ether und etwas Ethanol.

IR-Banden bei 3 600-2 100, 1 721, 1 676, 1 508, 1 324, 1 267, 1 182, 1 012 und 736 $cm^{-1}$ (in Nujol).

NMR-Signale bei δ = 9,18 (D ; J = 7,5 Hz ; 1H), 7,86 (S ; 1H) ; 7,84 (D ; J = 1,5 Hz ; 1H), 7,55 (D ; J = 5 Hz ; 1H), 7,22 (D ; J = 4 Hz ; 1H), 7,1 (Q ; J = 5 Hz ; $J_2$ = 4 Hz ; 1H), 6,9 (D ; J = 4 Hz ; 1H), 6,65 (Q ; $J_1$ = 4 Hz ; $J_2$ = 1,5 Hz ; 1H), 5,78 (D ; J = 7,5 Hz ; 1H), 3,95 (S ; 4H) (in DMF-$d_7$).

Vergleichsbeispiel 1.4

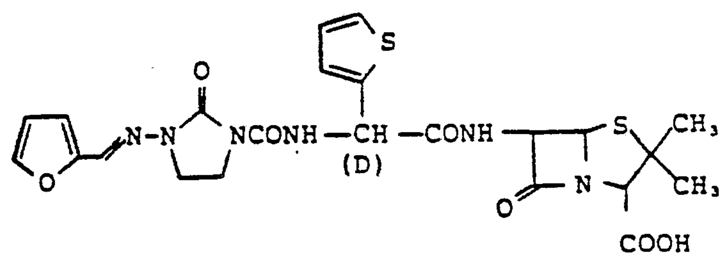

1 Mol (362 g + 15 g ; 96 %iges Produkt) Präcursäure von 1.3. wurde in 5,5 l Aceton suspendiert, bei Raumtemperatur mit 1 Mol (140 mo) Triethylamin versetzt und so lange (ca. 10 Minuten) gerührt, bei eine klare Lösung entstanden war. Man kühlte nun auf —40 °C Innentemperatur und fügte dabei 4,5 ml 3-Dimethylaminopropanol zu. Bei —40° wurden 1,05 Mol (100 ml) Chlorameisensäureethylester zugefügt und dann 15 Minuten bei —35 bis —30 °C (Innentemperatur) gerührt. Anschließend kühlte man auf —55° Innentemperatur.

Inzwischen bereitete man aus 1,05 Mol (227 g) 6-Amino-penicillansäure in 800 ml Wasser durch Zugabe von 2 n NaOH bis maximal pH = 7,8 eine Lösung von 6-APS-Na-Salz, verdünnte mit 1 l Aceton und kühlte auf —10 °C (eben noch keine Eisbildung).

Diese Lösung wurde zur —55 °C kalten Mischung des gemischten Anhydrids in einem Guß so rasch wie möglich zugegossen, wobei die Innentemperatur auf —35 bis —33° stieg. Das Kältebad wurde entfernt und der Kolben zum Schluß mit Wasser erwärmt, so daß nach ca. 50 Minuten + 12° Innentemperatur erreicht waren. Nun wurden 3 l Wasser zugefügt, das Aceton am Rotationsverdampfer im Vakuum abgezogen, nochmals 6,5 l Wasser und 125 g Kieselgur zugefügt, pH = 7 eingestellt, 15 Min. gerührt und abgesaugt (Zentrifugieren sollte besser sein, da das Absaugen bei einigen Ansätzen mehrere Stunden dauerte). Die wäßrige Phase wurde nun vorsichtig mit 1 n HCl angesäuert, wobei nach Erreichen von pH 4,5-5 Impfkristalle von D-Penicillinsäure zugesetzt wurden und einige Minuten zur Kristallbildung gerührt wurde, ehe man weiter auf pH 2 ansäuerte. Die Suspension wurde 20 Minuten nachgerührt und dann das weitgehend kristalline Produkt scharf abgesaugt. Das feuchte Rohprodukt wurde ausgewogen und unter Annahme einer 90 %igen Ausbeute (505 g) mit Wasser bis zu einer Gesamtmenge von 2 240 ml Wasser versetzt. Nun fügte man 5 l Aceton zu, löste mit 2 n NaOH bei pH = 7 und säuerte rasch wieder auf pH = 2 mit 1 n HCl an ; innerhalb weniger Sekunden begann das Produkt auszukristallisieren. Nach 2 Stunden Stehen im Eisbad wurde abgesaugt mit Wasser gewaschen und im Vakuum-Trockenschrank über $P_2O_5$ getrocknet.

Ausbeute : 64 % (375 g).

Das Produkt zeigte nach NMR-Spektrum ein D : L-Verhältnis von > 95 : < 5. Aus der Mutterlauge ließ sich durch Zugabe von viel Wasser, Abdekantieren und Durcharbeiten bzw. Rühren über Nacht mit frischem Wasser in 10-30 % Ausbeute Penicillinsäure mit einem D : L-Verhältnis von ca. 40 : 60 erhalten.

1.5.

Liegt der Gehalt des Produktes bei unter 95 % D und über 5 % L, so läßt sich wie folgt reinigen : (Bis zu einem D : L-Verhältnis von 4 : 1 durchführbar).

850 g (I) mit D : L = 90 : 10 wurden in 3,8 l $H_2O$ + 3,8 l Aceton mit 2 n NaOH bei pH = 7 unter Wasserkühlung (Innentemperatur ≤ 20 °C) in Lösung gebracht, mit 150 g Kieselgur 15 Minuten verrührt, abgesaugt und mit weiteren 3,8 l Aceton versetzt. Man säuerte nun auf pH = 2 mit 1 n HCl an, impfte an, ließ 2 Stunden in Eis unter gelegentlichem Aufrühren stehen, saugte ab, wusch mit Wasser und trocknete im Vakuum-Trockenschrank.

Ausbeute : 672 g (D : L nach NRM-Spektrum 98,5 : 1,5).

Aus der Mutterlauge wurden durch Zusatz von viel Wasser, Abdekantieren und Aufrühren über Nacht weitere 55 g Penicillinsäure (D : L = 63 : 37) enhalten.

Vergleichsbeispiel 2

377 g kristallisierte Penicillinsäure aus Vergleichsbeispiel 1 wurden in 1 500 ml Wasser + 450 ml Ethanol suspendiert und mit 1 n NaOH bei pH 7,0-7,5 in Lösung gebracht (falls nötig wird über G4-Fritte abgesaugt). Anschließend fügte man 6 l i-Propanol zu, und ließ 45 Minuten rühren. Es wurde scharf abgesaugt, mit i-Propanol suspendiert, erneut abgesaugt und 24 Stunden bei 40° im Trockenschrank über $P_2O_5$ getrocknet. 318 g (70,5 % Ausbeute, berechnet auf Na-Salz · 5 $H_2O$). Gehalt an i-PrOH 1-2 % der auch durch 4 tägiges weiteres Trocknen im Umluftschrank nicht gesenkt wurde.

DC (Butylacetat, Butanol, Eisessig, Phosphatpuffer pH = 7) praktisch einheitlich.

IR-Banden bei 3 650-2 500, 1 764, 1 725, 1 660, 1 597, 1 528, 1 512, 1 271, 1 234, 1 161, 1 018, 742 und 718 $cm^{-1}$ (in Nujol).

NMR-Signale bei δ = 7,42 (S ; 1H), 7,3 (D ; J = 5 Hz, 1H), 7,25 (D ; J ≈ 1 Hz ; 1H), 7,06 (D ; J = 4 Hz ; 1H), 6,9 (T ; J ≈ 4-5 Hz ; 1H), 6,4 (D ; J = 4 Hz ; 1H), 6,2 (Q ; $J_1$ ≈ 4 Hz ; $J_2$ ≈ 1 Hz ; 1H), 5,5 (S ; 1H), 5,35 (S ; 2H), 4,05 (S ; 1H), 3,65-3,25 (M ; 4H), 1,38 + 1,3 (D ; 6H) (in $D_2O$).

Aus der Mutterlauge ließen sich durch Verdünnen mit viel Wasser, Ansäuren auf pH = 2, Absaugen, Waschen mit Wasser und Trocknen im Exisiccator über $P_2O_5$ 44 g Säure regenerieren, die nach Umkristallisation erneut eingesetzt werden können, so daß die Gesamtausbeute bezogen auf verbrauchte Säure auf ~ 75 % steigt.

Beispiel 1

Kristallines Natriumsalz der D-6-{α-[(2-Oxo-3-furfuryliden-amino-imidazolidin-1-yl)-carbonylamino]-thienyl-2-acetamido}-penicillansäure, hergestellt aus amorphem Natriumsalz.

462 g des amorphen, nach Vergleichsbeispiel 2 hergestellen Natriumsalzes der D-6-{α-[2-Oxo-3-furfuryliden-amino-imidazolidin-1-yl)-carbonylamino]-thienyl-2-acetamido}-penicillansäure wurden portionsweise in einer Mischung aus 810 ml Wasser und 1 000 ml Ethanol gelöst und dann mit 5 l Ethanol unter Rühren bei Raumtemperatur versetzt. Man impfte an und fügte nach 30 Minuten Rühren weitere 1,1 l Ethanol zu, rührte 1 Stunde nach und ließ über Nacht stehen.

Man saugte nun scharf ab und trocknete im Umluftschrank bis zur Gewichtskonstanz.

Ausbeute : 64 % der Theorie. IR-Spektrum siehe Anhang Nr. 3.

NMR-Signale bei δ = 9,36 (1H), 9,23 (1H), 7,8 (2H), 7,5 (1H), 7,25 (1H), 7,05 (1H), 6,89 (1H), 6,62 (1H), 6,2 (1H), 5,74 (1H), 5,56 (1H), 4,35 (1H), 3,92 (4H), 1,6 (3H) und 1,5 ppm (3H) (in DMF-$d_7$).

Das Spektrum ist identisch mit dem des Produktes von Vergleichsbeispiel 2 im gleichen Lösungsmittel. 15 %ige Lösungen in Wasser bleiben bei Raumtemperatur 24 Stunden klar und gelieren nicht.

Beispiel 2

Kristallines Natriumsalz der D-6-{α-[2-Oxo-furfuryliden-amino-imidazolidin-1-yl)-carbonylamino]-thienyl-2-acetamido}-penicillansäure aus kristalliner Säure des Vergleichsbeispiels 1.

112 g (0,2 Mol) D-6-{α-[2-Oxo-3-furfurylidenamino-imidazolidin-1-yl)-carbonylamino]-thienyl-2-acetamido}-penicillansäure (hergestellt nach Vergleichsbeispiel 1) werden in 70 ml $H_2O$ + 240 ml Ethanol suspendiert und mit Mischung aus 96 ml 2 n NaOH + 96 ml EtOH auf pH = 7,5 eingestellt. Die klare Lösung wird mit 1 420 ml Ethanol versetzt ; man läßt die ausgefallenen dunklen Flocken 10 Minuten absitzen, dekantiert über G 4 Glasfilternutsche und saugt auch die abgesetzte Suspension ab. Anschließend wird die Lösung angeimpft und magnetisch schnell gerührt. Nach 30 Minuten versetzt man mit 200 ml Ethanol, rührt 7 Stunden, versetzt nochmals mit 300 ml Ethanol, rührt über Nacht, saugt scharf ab und wäscht mit Ethanol nach. Getrocknet wird über $P_2O_5$ im Vakuum-Exsiccator.

Ausbeute : 60-73 % IR und NMR-Spektrum stimmen mit denen des Beispiel 1 überein.

**Patentansprüche**

1. Natriumsalz der D-6-{α-[(2-Oxo-3-furfurylidenamino-imidazolidin-1-yl)-carbonylamino]-thienyl-2-acetamido}-penicillansäure der Formel (I)

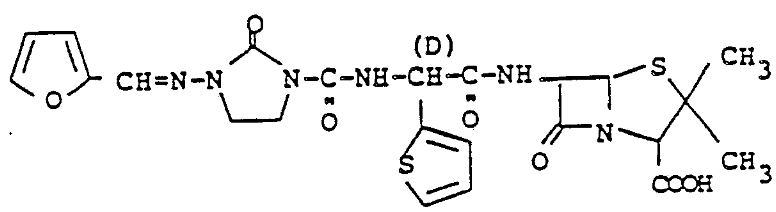

(I)

in reiner kristalliner Form, gekennzeichnet durch NMR Signale bei δ = 9,36 (1H), 9,23 (1H), 7,8 (2H), 7,5

(1H), 7,25 (1H), 7,05 (1H), 6,89 (1H), 6,62 (1H), 6,2 (1H), 5,74 (1H), 5,56 (1H), 4,35 (1H), 3,92 (4H), 1,6 (3H) und 1,5 ppm (3H) (in DMF-$d_7$).

2. Verfahren zur Herstellung des Natriumsalzes der Verbindung der Formel (I)

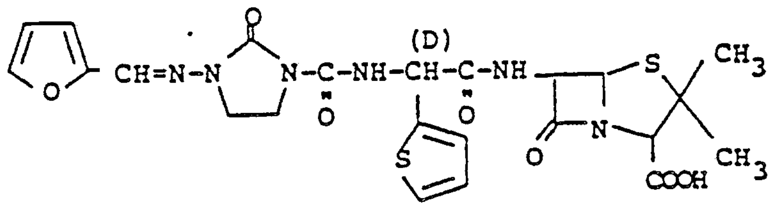

(I)

in reiner kristalliner Form, dadurch gekennzeichnet, daß man das amorphe Natriumsalz der D-Penicillansäure der Formel (I) aus einem Ethanol-Wasser-Gemisch in den Mengenverhältnissen von Ethanol : Wasser im Bereich von 5 : 1 bis 15 : 1 bei Temperaturen von 0 °C bis 50 °C kristallisiert.

3. Verfahren zur Herstellung des Natriumsalzes der D-Penicillansäure der Formel (I) gemäß Anspruch 2 in reiner kristalliner Form, dadurch gekennzeichnet, daß man die kristalline Säure der Formel (I) in das Natriumsalz überführt und dieses gemäß Anspruch 2 aus Ethanol-Wasser-Gemischen kristallisiert.

4. Arzneimittel, enthaltend das kristalline Natriumsalz der D-Penicillansäure der Formel (I) gemäß Anspruch 1 als Wirkstoff.

5. Verfahren zur Herstellung von Arzneimitteln gemäß Anspruch 4, dadurch gekennzeichnet, daß man den Wirkstoff gegebenenfalls unter Verwendung üblicher Hilfs- und Trägerstoffe in eine geeignete Applikationsform überführt.

6. Verwendung des Wirkstoffes gemäß Anspruch 1 bei dèr Herstellung von Arzneimitteln.

**Claims**

1. Sodium salt of D-6-{α-[(2-oxo-3-furfurylideneamino-imidazolidin-1-yl)-carbonylamino]-2-thienylacetamido}-penicillanic acid of the formula (I)

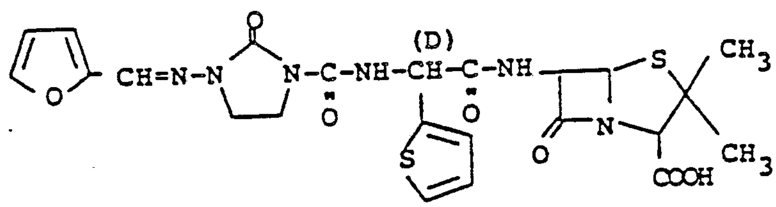

(I)

in pure crystalline form, characterized by the following NMR signals : δ = 9.36 (1H), 9.23 (1H), 7.8 (2H), 7.5 (1H), 7.25 (1H), 7.05 (1H), 6.89 (1H), 6.62 (1H), 6.2 (1H), 5.74 (1H), 5.56 (1H), 4.35 (1H), 3.92 (4H), 1.6 (3H) and 1,5 ppm (3H) (in DMF-$d_7$).

2. Process for the preparation of the sodium salt of the compound of the formula (I)

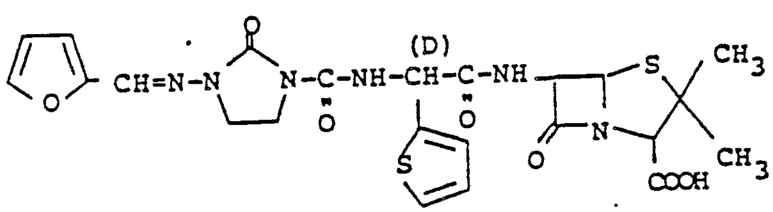

(I)

in pure crystalline form, characterized in that the amorphous sodium salt of the D-penicillanic acid of the formula (I) is crystallized from an ethanol/water mixture in quantitative ratios of ethanol : water in the range from 5 : 1 to 15 : 1 at temperatures from 0 °C to 50 °C.

3. Process for the preparation of the sodium salt of the D-penicillanic acid of the formula (I) according to Claim 2 in pure crystalline form, characterized in that the crystalline acid of the formula (I) is converted into the sodium salt and this is crystallized from ethanol/water mixtures according to Claim 2.

4. Medicaments containing the crystalline sodium salt of the D-penicillanic acid of the formula (I) according to Claim 1, as the active compound.

5. Process for the preparation of medicaments according to Claim 4, characterized in that the active compound is converted into a suitable administration form, if appropriate using customary auxiliaries and

excipients.

6. Use of the substance according to Claim 1 in the preparation of medicaments.

## Revendications

1. Sel de sodium de l'acide D-6-{α-[(2-oxo-3-furfurylidène-amino-imidazolidine-1-yl)-carbonylamino]-thiényl-2-acétamido}-pénicillanique de formule (I)

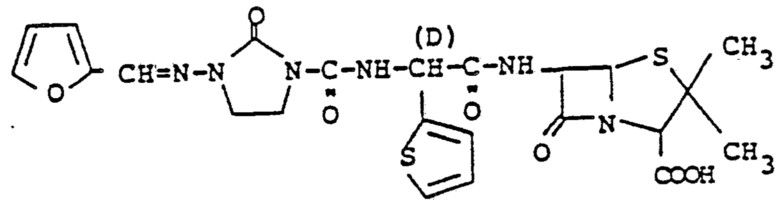

(I)

sous la forme cristalline pure, caractérisé par des signaux de résonance magnétique nucléaire pour δ = 9,36 (1H), 9,23 (1H), 7,8 (2H), 7,5 (1H), 7,25 (1H), 7,05 (1H), 6,89 (1H), 6,62 (1H), 6,2 (1H), 5,74 (1H), 5,56 (1H), 4,35 (1H), 3,92 (4H), 1,6 (3H) et 1,5 ppm (3H) (dans DMF-$d_7$).

2. Procédé de préparation du sel de sodium du composé de formule (I)

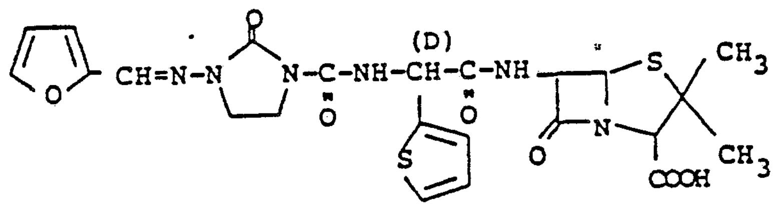

(I)

sous la forme cristalline pure, caractérisé en ce qu'on fait cristalliser à des températures de 0 à 50 °C le sel de sodium amorphe de l'acide D-pénicillanique de formule (I) dans un mélange d'éthanol et d'eau dans des rapports quantitatifs de l'éthanol à l'eau compris dans l'intervalle de 5 : 1 à 15 : 1.

3. Procédé de préparation du sel de sodium de l'acide D-pénicillanique de formule (I) suivant la revendication 2, sous la forme cristalline pure, caractérisé en ce qu'on transforme l'acide cristallin de formule (I) en le sel de sodium que l'on fait cristalliser conformément à la revendication 2 dans des mélanges d'éthanol et d'eau.

4. Médicament, contenant le sel de sodium cristallin de l'acide D-pénicillanique de formule (I) suivant la revendication 1 comme substance active.

5. Procédé de préparation de médicaments suivant la revendication 4, caractérisé en ce qu'on fait prendre à la substance active une forme d'application appropriée, en utilisant le cas échéant des adjuvants et des supports classiques.

6. Utilisation de la substance active suivant la revendication 1 dans la préparation de médicaments.

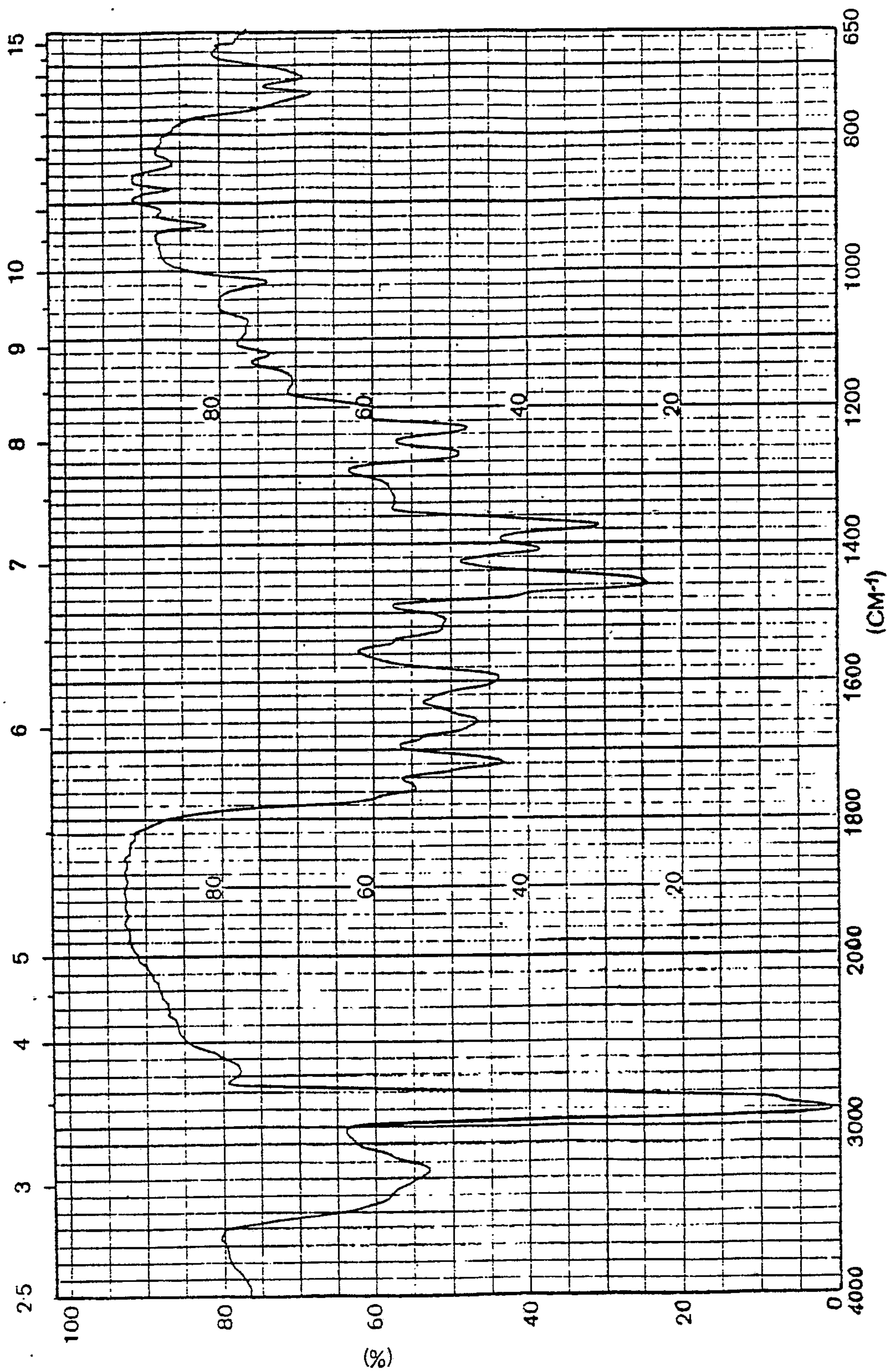


FIG. 1

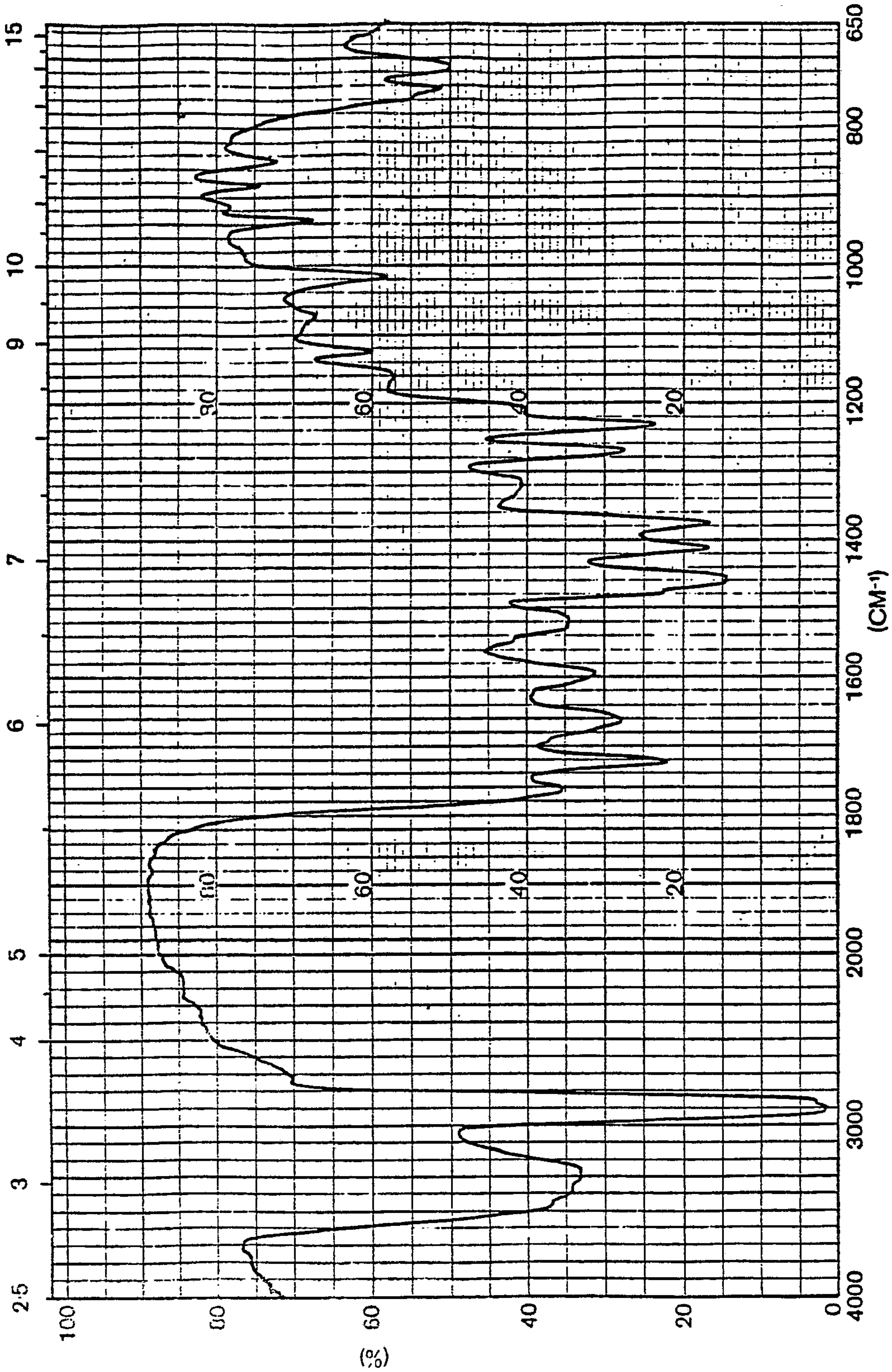
2.5
3
4
5
6
7
9
10
15
4000
3000
2000
1800
1600
1400
1200
1000
800
650
(CM-1)
100
80
60
40
20
0
(%)

FIG. 2

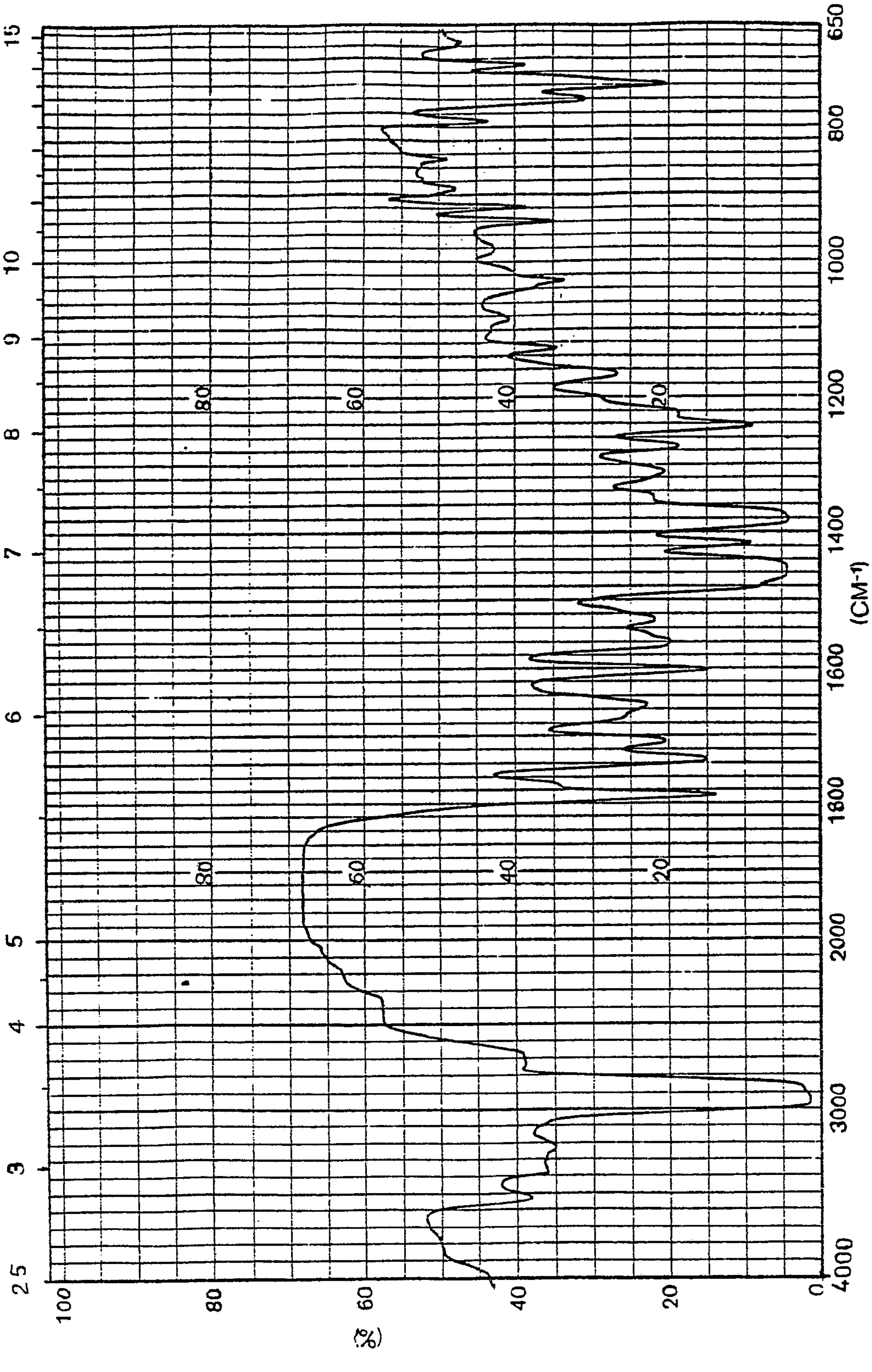
2.5
3
4
5
6
7
8
9
10
15
4000
3000
2000
1800
1600
1400
1200
1000
800
650
(CM-1)
100
80
60
40
20
0
(%)

FIG. 3